Europäisches Patentamt

European Patent Office

Office européen des brevets

(11) Veröffentlichungsnummer : **0 083 050**
**B1**

(12) **EUROPÄISCHE PATENTSCHRIFT**

(45) Veröffentlichungstag der Patentschrift :
03.04.85

(21) Anmeldenummer : **82111783.5**

(22) Anmeldetag : **18.12.82**

(51) Int. Cl.⁴ : **A 43 C 15/09**, A 43 B 5/04,
A 61 F 5/01

(54) **Orthopädischer Sohlenuntersatz.**

(30) Priorität : **29.12.81 DE 3151721**

(43) Veröffentlichungstag der Anmeldung :
**06.07.83 Patentblatt 83/27**

(45) Bekanntmachung des Hinweises auf die Patenterteilung : **03.04.85 Patentblatt 85/14**

(84) Benannte Vertragsstaaten :
**AT CH LI**

(56) Entgegenhaltungen :
CH-A-  595 791
DE-C-  344 462
DE-C-  742 485
DE-U- 8 014 830
FR-A- 1 128 009
US-A- 3 786 579

(73) Patentinhaber : **Werne, Paul**
**Kaiserstrasse 69**
**D-7890 Waldshut-Tiengen 1 (DE)**

(72) Erfinder : **Werne, Paul**
**Kaiserstrasse 69**
**D-7890 Waldshut-Tiengen 1 (DE)**

(74) Vertreter : **Kluge, Heinz, Dr.-Ing.**
**Fluhmattstrasse 37**
**CH-5400 Baden (CH)**

Jouve, 18, rue St-Denis, 75001 Paris, France

EP 0 083 050 B1

## Beschreibung

Die vorliegende Erfindung bezieht sich auf eine Vorrichtung gemäss dem Oberbegriff des Anspruchs 1.

Schon bei Vierzigjährigen leiden etwa 50 % an Kniegelenkschäden. Die Kniearthrose wirkt sich insbesondere beim Bergabstieg sehr schmerzaft aus. Denn hierbei wird die Muskulatur des Oberschenkel-Kniestreckers stark angespannt, was zu einem erhöhten druck durch die Kniescheibe auf das Kniegelenk führt. Beim Bergaufstieg ist dies nicht der Fall.

Aus der CH-A-596 785 ist ein an einem Skistiefel lösbar angeordneter Sohlenuntersatz bekannt, welcher durch seine Formgebung die durch den Skistiefel, insbesondere einen starren Schalenskistiefel, aufgezwungene Beugung des Knies und damit dessen Belastung beim Stehen vermindern soll, um Gesundheitsschäden vorzubeugen. Der bekannte Sohlenuntersatz besteht aus einem einstückigen Laufsohlenteil aus elastischem Material, welcher einen näherungsweise trapezförmigen Abrollkörper im worderen Sohlenbereich und einen näherungsweise dreiecksförmigen Körper im hinteren Sohlenbereich aufweist.

Es ist Hauptaufgabe der Erfindung, normale Berg- und/oder Wanderschuhe mit einer orthopädischen Vorrichtung zu versehen, die selbst bei steilen Hägen (bis 35° Neigung) ein weitgehendes Muskelgleichgewicht zwischen Oberschenkel-Strecker und -Beuger gewährleistet.

Die Lösung dieser Aufgabe gelingt mit den Elementen, welche im Kennzeichen des Anspruchs 1 genannt sind.

Die erfindungsgemässe Vorrichtung unterscheidet sich von dem bekannten Laufsohlenteil durch zwei wesentliche Punkte. Zum einen wird durch den starren Grundkörper die beim normalen Berg- und/oder Wanderschuh flexible Sohle versteift, so dass der Fuss nicht mehr über seinen Ballen abrollen kann, wie es die bekannte Vorrichtung gerade in Verbindung mit einem starren Skistiefel ermöglichen soll. Zum anderen ist der Scheitelpunkt des Sperrkörpers soweit vor dem Ballen angeordnet, dass eine Verbindungsgerade Scheitelpunkt — Kniegelenk durch das erste Grosszehengelenk verläuft, wenn die Ebene des Grundkörpers, also die Fläche, auf der der Schuh steht, etwa horizontal liegt. Diese Ausbildung bewirkt nach dem Auftreten eine starke Rückhebelwirkung auf das Kniegelenk, wodurch dieses vor dem nächsten Abwickeln kurzfristig einmal in Streckstellung kommt. Dabei werden speziell die belastungs- und schmerzempfindlichen Kniegelenksbereiche immer wieder entlastet.

In den Zeichnungen sind Ausführungsbeispiele des Erfindungsgegenstandes vereinfacht wiedergegeben.

Es zeigt

Figur 1   einen starren Grundkörper der orthopädischen Vorrichtung in Draufsicht.

Figur 2   den starren Grundkörper teilweise perspektivisch in Seitenansicht.

Figur 3   einen Teil des starren Grundkörpers ohne Abkröpfung.

Figur 4   die am Schuh angebrachte orthopädische Vorrichtung.

Figur 5a   schematisch die Belastung des Kniegelenks beim Bergabstieg.

Figur 5b   schematisch die Entlastung des Kniegelenks mit Hilfe der orthopädischen Vorrichtung.

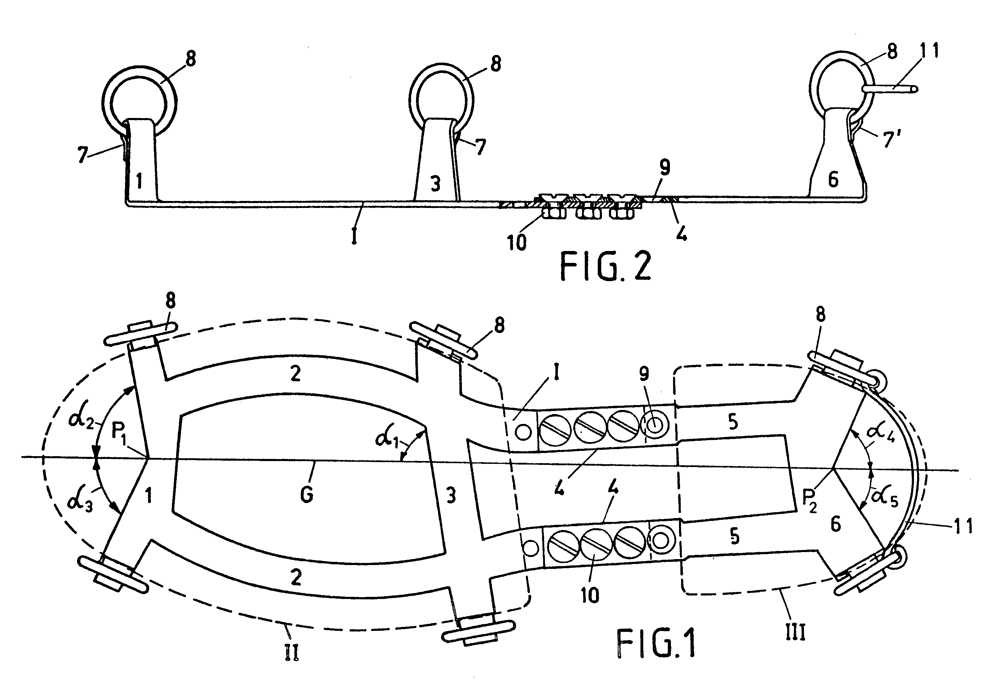

In Fig. 1 ist mit I ein starrer Grundkörper der orthopädischen Vorrichtung bezeichnet. Gestrichelt angedeutet sind ein Sperrkörper II und ein Absatzkeil III. Der Grundkörper I wird im wesentlichen gebildet aus einem winkelförmigen Zehensteg 1, einem ellipsenförmigen Sohlenteil 2, einem überwiegend geradlinigem Ballensteg 3, einem geradlinigen Verstellteil 4, einem ebensolchen Absatzteil 5 sowie einem winkelförmigen Absatzsteg 6. Eine Gerade G zwischen dem Schnittpunkt $P_1$ der Schenkel des Zehenstegs 1 und dem Schnittpunkt $P_2$ der Schenkel des Absatzstegs 6 bildet mit den Stegen 3, 1, 6 Winkel $\alpha_1$, $\alpha_2$, $\alpha_3$, $\alpha_4$ und $\alpha_5$. Es hat sich gezeigt, dass eine sehr gute Stabilität beim Auftreten gewährleistet ist, wenn sowohl $\alpha_2$ grösser ist als $\alpha_3$, als auch $\alpha_4$ grösser ist als $\alpha_5$ und $\alpha_1$ Werte zwischen 70° und 85° aufweist. Ferner ist es günstig, wenn der Grundkörper I aus Chromnickelstahl besteht.

Fig. 2 lässt noch deutlicher als Fig. 1 erkennen, dass die Stege 1, 3, 6 an ihren Enden abgekröpft sind und Befestigungslaschen 7, 7' bilden, in denen Halteringe 8 angeordnet sind. In diesen Halteringen werden nicht gezeigte Riemen zur Befestigung der orthopädischen Vorrichtung am Schuh eingezogen. Die beiden Halteringe 8 des Absatzstegs 6 sind mittels eines Sicherungsringes 11 miteinander verbunden. Der Verstellteil 4 weist Bohrungen 9 und Schraubverbindungen 10 auf.

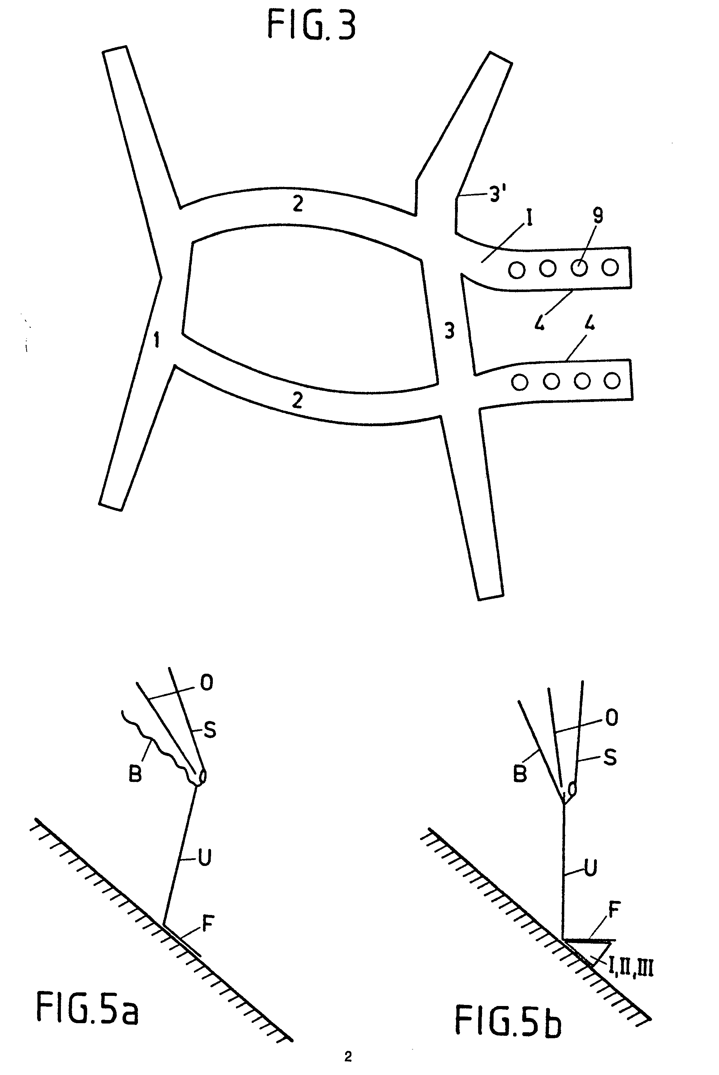

Fig. 3 zeigt nochmals teilweise den grundkörper I. Gleiche Teile sind mit denselben Bezugszeichen versehen wie in den vorhergehenden Figuren. Die Stege 1 und 3 sind noch nicht nach oben abgekröpft. Es lässt sich erkennen, dass der am Grosszehballen liegende Ballensteg 3 an der Stelle 3' zusätzlich abgewinkelt ist. Hierdurch lässt sich der Druck auf den Grosszehballen beträchtlich vermindern.

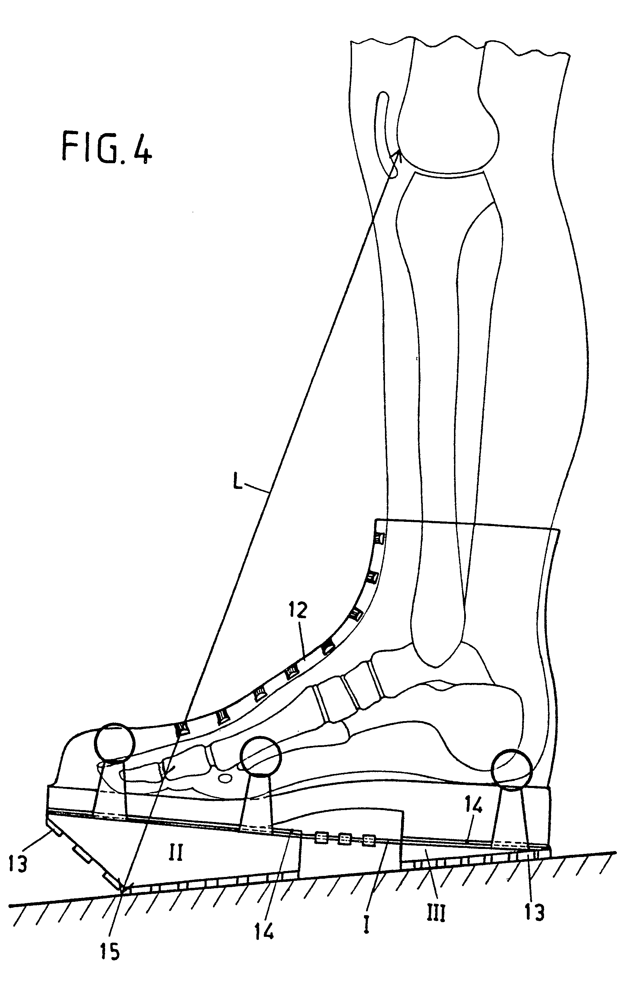

Fig. 4 gibt die orthopädische Vorrichtung an einem Schuh 12 wieder. Ein Fuss und ein Bein mit dem zugehörigen Knochengerüst sind angedeutet. Der Sperrkörper II hat näherungsweise Trapezform, der Absatzkeil III näherungsweise Dreiecksform. Die Teile II und III sind am Grundkörper I festgeklebt und mit Profilen 13 versehen. Ferner ist auf den Grundkörper I eine dünne Deckschicht 14 aufgeklebt. Das Femurogelenk des Beines wird dann mit Sicherheit entlastet, wenn eine (gedachte) Gerade L zwischen dem Scheitelpunkt 15 des Sperrkörpers II zum genannten Gelenk durch das erste Zehengelenk der Grosszehe verläuft. Als Material für den Sperrkörper II und den Absatzkeil III sowie die Deckschicht 14 hat sich Leichtzellgummi bewährt.

In den Figuren 5a und 5b bedeutet F den Fuss, U den Unterschenkel, O den Oberschenkelknochen, S die Muskulatur des Oberschenkelstreckers, B diejenige des Oberschenkelbeugers. Während gemäss Fig. 5a das Kniegelenk ausserordentlich belastet ist, zeigt Fig. 5b bei Verwendung der orthopädischen Vorrichtung I, II, III ein Muskelgleichgewicht zwischen Oberschenkelstrecker und -beuger und damit eine Entlastung des Kniegelenks.

Bezeichnungsliste

I Starrer Grundkörper
II Sperrkörper
III Absatzkeil
1 Winkelförmiger Zehensteg
2 Ellipsenförmiger Sohlenteil
3 überwiegend geradliniger Ballensteg
3' Abwinkelung am Ballensteg 3
4 Geradliniger Verstellteil
5 Geradliniger Absatzteil
6 Winkelförmiger Absatzsteg
7, 7' Befestigungslaschen der Stege 1, 3, 6
8 Halteringe
9 Bohrungen des Verstellteils 4
10 Schraubverbindungen des Verstellteils 4
11 Sicherungsring durch Halteringe 8 des Absatzstegs 6
12 Schuh
13 Profile am Sperrkörper II und Absatzkeil III
14 Deckschicht auf Grundkörper I
15 Scheitelpunkt des Sperrkörpers II
$P_1$ Schnittpunkt der Schenkel des Zehenstegs 1
$P_2$ Schnittpunkt der Schenkel des Absatzstegs 6
G Bezugsgerade zwischen $P_1$ und $P_2$
$\alpha_1$ Winkel zwischen G und innerem Teil des Ballenstegs 3
$\alpha_2, \alpha_3$ Teilwinkel zwischen G und den Schenkeln des Zehenstegs 1
$\alpha_4, \alpha_5$ Teilwinkel zwischen G und den Schenkeln des Absatzstegs 6
L Gerade Linie zwischen dem Scheitelpunkt 15 des Sperrkörpers II und dem Kniegelenk
F Fuss
U Unterschenkel
O Oberschenkelknochen
S Muskulatur des Oberschenkelstreckers
B Muskulatur des Oberschenkelbeugers.

**Ansprüche**

1. Orthopädische Vorrichtung, die an einem Schuh lösbar angeordnet ist, einen näherungsweise trapezförmigen Körper im vorderen Sohlenbereich und einen näherungsweise dreiecksförmigen Körper im hinteren Sohlenbereich aufweist, dadurch gekennzeichnet, dass der näherungsweise trapezförmiger Sperrkörper (II) und der näherungsweise dreiecksförmiger Absatzkeil (III) an einem starren Grundkörper (I) angebracht sind und eine gerade Linie (L) zwischen dem Scheitelpunkt (15) des Sperrkörpers (II) zum Kniegelenk durch das erste Zehengelenk der Grosszehe verläuft, wenn die Ebene des Grundkörpers etwa horizontal liegt.

2. Vorrichtung nach Anspruch 1, dadurch gekennzeichnet, dass der Grundkörper (I) einen winkelförmigen Zehensteg (1), einen ellipsenförmigen Sohlenteil (2), einen teilweise geradlinigen Ballensteg (3), einen geradlinigen Verstellteil (4) und Absatzteil (5) sowie einen winkelförmigen Absatzsteg (6) aufweist.

3. Vorrichtung nach Anspruch 2, dadurch gekennzeichnet, dass die Stege (1, 3, 6) an ihren Enden als nach oben abgekröpfte Befestigungslaschen (7, 7') ausgebildet sind, welche Halteringe (8) aufnehmen.

4. Vorrichtung nach Anspruch 3, dadurch gekennzeichnet, dass die Befestigungslaschen (7') des Absatzstegs (6) die Laschen (7) der anderen Stege (1, 3) überragen.

5. Vorrichtung nach Anspruch 3 und 4, dadurch gekennzeichnet, dass ein Sicherungsring (11) die Halteringe (8) des Absatzsteges (6) miteinander verbindet.

6. Vorrichtung nach Anspruch 2, dadurch gekennzeichnet, dass der Ballensteg (3) an der Fussinnenseite in noch nicht abgekröpfter Lage nach hinten abgewinkelt ist.

7. Vorrichtung nach Anspruch 2, dadurch gekennzeichnet, dass der Verstellteil (4) Bohrungen (9) und Schraubverbindungen (10) aufweist.

8. Vorrichtung nach Anspruch 2, dadurch gekennzeichnet, dass der innere Teil des Ballenstegs (3) gegenüber einer Bezugsgeraden (G) zwischen dem Schnittpunkt ($P_1$) der Schenkel des Zehenstegs (1) und dem Schnittpunkt ($P_2$) der Schenkel des Absatzstegs (6) einen Winkel ($\alpha_1$) zwischen 70° und 85° aufweist.

9. Vorrichtung nach Anspruch 8, dadurch gekennzeichnet, dass die zwischen der Bezugsgeraden (G) und den Schenkeln der Winkelstege (1, 6) gebildeten ersten und zweiten Teilwinkel ($\alpha_2$, $\alpha_3$; $\alpha_4$, $\alpha_5$) eine unterschiedliche Grösse aufweisen.

10. Vorrichtung nach Anspruch 9, dadurch gekennzeichnet, dass der erste Teilwinkel ($\alpha_2$) des Zehenstegs (1) grösser ist als dessen zweiter Teilwinkel ($\alpha_3$), und dass der erste Teilwinkel ($\alpha_4$) des Absatzstegs (6) grösser ist als dessen zweiter Teilwinkel ($\alpha_5$).

11. Vorrichtung nach Anspruch 1, dadurch gekennzeichnet, dass der Grundkörper (I) aus Chromnickelstahl besteht.

12. Vorrichtung nach Anspruch 1, dadurch gekennzeichnet, dass sowohl der Sperrkörper (II) als auch der Absatzkeil (III) laufseitig mit Profilen (13) versehen sind.

13. Vorrichtung nach Anspruch 1, dadurch gekennzeichnet, dass der Sperrkörper (II), der Absatzkeil (III) und eine Deckschicht (14) am Grundkörper (I) angeklebt sind.

14. Vorrichtung nach Anspruch 1, dadurch gekennzeichnet, dass sowohl der Sperrkörper (II) als auch der Absatzkeil (III) und die Deckschicht (14) aus Leichtzellgummi bestehen.

## Claims

1. Orthopaedic device which is releasably arranged on a shoe and comprises an approximately trapezoidal body in the front region of the sole and an approximately triangular body in the rear region of the sole, characterised in that the approximately trapezoidal blocking body (II) and the approximately triangular heel wedge (III) are fitted to a rigid base body (I), an a straight line (L) runs between the vertex (15) of the blocking body (II) and the knee joint through the first toe joint of the big toe, when the plane of the base body is approximately horizontal.

2. Device according to Claim 1, characterised in that the base body (I) comprises an angular toe ridge (1), an elliptical sole part (2), a partially straight ball ridge (3), a straight adjusting part (4) and heel part (5) as well as an angular heel bridge (6).

3. Device according to Claim 2, characterised in that the ridges (1, 3, 6) are formed at their ends as fixing lugs (7, 7') which are bent off upwards and receive holder rings (8).

4. Device according to Claim 3, characterised in that the fixing lugs (7') of the bridge (6) project to a greater height than the lugs (7) of the other bridges (1, 3).

5. Device according to Claim 3 and 4, characterised in that a securing ring (11) mutually connects the holder rings (8) of the heel bridge (6).

6. Device according to Claim 2, characterised in that the ball bridge (3) is angled rearwards, where it is not yet bent off, on the inside of the foot.

7. Device according to Claim 2, characterised in that the adjustment part (4) contains holes (9) and screw connections (10).

8. Device according to Claim 2, characterised in that the inner part of the ball bridge (3) has an angle ($\alpha_1$) of between 70° and 85° relative to a reference straight line (G) between the point of intersection ($P_1$) of the arms of the toe bridge (1) and the point of intersection ($P_2$) of the arms of the heel bridge (6).

9. Device according to Claim 8, characterised in that the first and second part angles ($\alpha_2$, $\alpha_3$ ; $\alpha_4$, $\alpha_5$) formed between the reference straight line (G) and the arms of the angle bridges (1, 6) are of different magnitude.

10. Device according to Claim 9, characterised in that the first part angle ($\alpha_2$) of the toe bridge (1) is greater than the second part angle ($\alpha_3$) of the latter, and that the first part angle ($\alpha_4$) of the heel bridge (6) is greater than the second part angle ($\alpha_5$) of the latter.

11. Device according to Claim 1, characterised in that the base body (I) consists of chrome-nickel steel.

12. Device according to Claim 1, characterised in that both the blocking body (II) and the heel wedge (III) are provided with profiles (13) on the walking side.

13. Device according to Claim 1, characterised in that the blocking body (II), the heel wedge (III) and a cover layer (14) are glued to the base body (I).

14. Device according to Claim 1, characterised in that the blocking body (II) as well as the heel wedge (III) and the cover layer (14) consist of light-weight cellular rubber.

## Revendications

1. Dispositif orthopédique qui est monté de manière amovible sur une chaussure et qui présente un corps approximativement trapézoïdal dans la zone antérieure de la semelle et un corps approximativement triangulaire dans la zone postérieure de la semelle, caractérisé en ce que le corps d'arrêt approximativement trapézoïdal (II) et le coin de talon approximativement triangulaire (III) sont montés sur un corps de base rigide (I) et une ligne droite (L) reliant le sommet (15) du corps d'arrêt (II), et l'articulation du genou passe par la première articulation du gros orteil lorsque le plan du corps de base est à peu près horizontal.

2. Dispositif suivant la revendication 1, caractérisé en ce que le corps de base (I) comporte une bande transversale angulaire (1) pour les orteils, une partie de semelle ellipsoïdale (2), une bande transversale d'éminence métatarsienne partiellement droite (3), une partie de réglage droite (4) et une partie de talon (5) ainsi qu'une bande transversale de talon angulaire (6).

3. Dispositif suivant la revendication 2, caractérisé en ce que les bandes transversales (1, 3, 6) présentent des extrémités qui ont la forme de tirants de fixation (7, 7') coudés vers le haut qui reçoivent des anneaux de retenue (8).

4. Dispositif suivant la revendication 3, caractérisé en ce que les tirants de fixation (7') de la bande transversale de talon (6) dépassent les tirants (7) des autres bandes transversales (1, 3).

5. Dispositif suivant les revendications 3 et 4, caractérisé en ce qu'un anneau de sûreté (11) relie l'un à l'autre les anneaux de retenue (8) de la bande transversale de talon (6).

6. Dispositif suivant la revendication 2, caractérisé en ce que la bande transversale de l'éminence métatarsienne (3) est inclinée vers l'arrière au niveau du côté intérieur du pied dans l'état non encore coudé.

7. Dispositif suivant la revendication 2, caractérisé en ce que la partie de réglage (4) présente des forures (9) et des assemblages à vis (10).

8. Dispositif suivant la revendication 2, caractérisé en ce que la partie interne de la bande transversale (3) de l'éminence métatarsienne forme un angle ($\alpha_1$) compris entre 70 et 85° par rapport à une droite de référence (G) tracée entre le point d'intersection ($P_1$) des ailes de la bande transversale (1) des orteils et le point d'intersection ($P_2$) des ailes de la bande transversale (6) du talon.

9. Dispositif suivant la revendication 8, caractérisé en ce que les premiers et seconds angles partiels ($\alpha_2$, $\alpha_3$ ; $\alpha_4$, $\alpha_5$) formés entre la ligne droite de référence (G) et les ailes des bandes angulaires (1, 6) présentent des valeurs différentes.

10. Dispositif suivant la revendication 9, caractérisé en ce que le premier angle partiel ($\alpha_2$) de la bande transversale (1) des orteils est supérieur à son second angle partiel ($\alpha_3$) et le premier angle partiel ($\alpha_4$) de la bande transversale de talon (6) est supérieur à son second angle partiel ($\alpha_5$).

11. Dispositif suivant la revendication 1, caractérisé en ce que le corps de base (I) est en acier au nickel-chrome.

12. Dispositif suivant la revendication 1, caractérisé en ce que le corps d'arrêt (II), de même que le coin de talon (III) sont pourvus de profils (13) du côté de marche.

13. Dispositif suivant la revendication 1, caractérisé en ce que le corps d'arrêt (II), le coin de talon (III) et une couche de recouvrement (14) sont collés sur le corps de base (I).

14. Dispositif suivant la revendication 1, caractérisé en ce que le corps d'arrêt (II), de même que le coin de talon (III) et la couche de recouvrement (14) sont en caoutchouc cellulaire léger.

FIG. 2

FIG.1

0 083 050

# FIG.3

# FIG.5a

# FIG.5b

FIG.4

L

12

13

II

15

14

I

III

14

13